# EUROPÄISCHE PATENTSCHRIFT

(11) **EP 4 298 275 B1**
(45) Veröffentlichungstag und Bekanntmachung des Hinweises auf die Patenterteilung: **30.04.2025**
(21) Anmeldenummer: 21840041.4
(22) Anmeldetag: 21.12.2021
(51) Int. Cl.: D03D 11/02, D03D 15/56, D03D 1/00, D03D 15/533

(54) **TEXTILES FLACHMATERIAL MIT INTEGRIERTEN TASCHEN ZUR AUFNAHME VON ELEKTRISCHEN UND/ODER ELEKTRONISCHEN BAUTEILEN FÜR MEDIZINTECHNISCHE MESS- UND/ODER STEUERZWECKE**
FLAT TEXTILE MATERIAL HAVING INTEGRATED POCKETS FOR RECEIVING ELECTRICAL AND/OR ELECTRONIC COMPONENTS FOR MEDICAL MEASUREMENT AND/OR CONTROL PURPOSES
MATÉRIAU TEXTILE PLAT À POCHES INTÉGRÉES DESTINÉES À RECEVOIR DES COMPOSANTS ÉLECTRIQUES ET/OU ÉLECTRONIQUES À DES FINS DE MESURE ET/OU DE COMMANDE D'ORDRE MÉDICAL

(30) Priorität: 24.02.2021 DE 102021104437
(43) Veröffentlichungstag der Anmeldung: 03.01.2024
(73) Patentinhaber: KOB GmbH, 67752 Wolfstein (DE)
(72) Erfinder: BORCZYK, Sina, 67655 Kaiserslautern (DE); MEYER, Marcin, 67752 Wolfstein (DE)
(74) Vertreter: DREISS Patentanwälte PartG mbB
(86) Internationale Anmeldenummer: PCT/EP2021/086944
(87) Internationale Veröffentlichungsnummer: WO 2022/179740

(56) Entgegenhaltungen:
- WO-A2-2017/030851
- FR-A1- 3 054 247
- US-A1- 2020 087 823
- US-A1- 2020 283 935

## Beschreibung

Die Erfindung betrifft ein textiles Flachmaterial zum Anlegen an einen menschlichen oder tierischen Körper und zur Aufnahme von elektrischen und/oder elektronischen Bauteilen für medizintechnische Mess- und/oder Steuerzwecke.

Aus der US 2021/0118427 A1 sind mehrlagige textile Flachmaterialien mit Kavitäten zur Aufnahme von elektronischen Steuereinheiten bekannt, wobei die elektronischen Steuereinheiten in ein Gewebe eingebettet sind und Kontaktstellen umfassen, welche mit an der Kavität vorgesehenen und in das Gewebe eingebundenen elektrisch leitfähigen Fäden zur Übertragung von elektrischen Signalen zusammenwirken.

FR 3 054 247 A1 offenbart ein textiles Flachmaterial zum Anlegen an einen menschlichen oder tierischen Körper zur Ausübung einer Sensorfunktion, nämlich zur Detektion einer Druckausübung. Es umfasst: eine Längsrichtung, eine quer zur Längsrichtung verlaufende Querrichtung, eine Erstreckungsebene, eine Dickenrichtung orthogonal zur Erstreckungsebene, wenigstens einen sich in der Längsrichtung erstreckenden Trägerabschnitt mit einer Trägerlage, wenigstens einen sich in der Längsrichtung erstreckenden und in der Längsrichtung an den Trägerabschnitt anschließenden Taschenabschnitt mit wenigstens einer Tasche, wobei die wenigstens eine Tasche eine körperzugewandte erste Taschenlage und eine körperabgewandte zweite Taschenlage umfasst, die zwischen sich einen Taschenraum begrenzen, wobei die wenigstens eine Tasche allseits umschlossen oder alternativ dazu eine Taschenöffnung aufweisen kann. Das textile Flachmaterial umfasst in der Längsrichtung angeordnete Kettfäden und in der Querrichtung angeordnete Schussfäden, welche die Trägerlage, die erste Taschenlage und die zweite Taschenlage bilden. In der Längsrichtung und in der Querrichtung ist jeweils wenigstens ein elektrischer Leiter in das textile Flachmaterial eingewoben, wobei die in Längsrichtung verlaufenden elektrischen Leiter in der ersten Taschenlage und die in Querrichtung verlaufenden elektrischen Leiter in der zweiten Taschenlage eingewoben und somit gehalten sind. Die Taschenlagen sind derart konfiguriert, dass sie im unbelasteten Zustand des textilen Flachmaterials samt der darin gehaltenen Leiter in der Dickenrichtung auf Abstand zueinander angeordnet sind. Wenn eine Druckkraft orthogonal zur Erstreckungsebene des textilen Flachmaterials im Bereich der Taschen ausgeübt wird, so wird ein Kontakt der in Längsrichtung und der in Querrichtung verlaufenden elektrischen Leiter hergestellt und die Druckkraftbelastung kann so detektiert werden. Hierfür beträgt ein Flottierungsabstand der elektrischen Leiter im Zentrum einer jeweiligen Tasche zur Bildung eines Kontaktbereichs zwischen 1 mm bis höchstens 2 mm.

Der vorliegenden Erfindung liegt die Aufgabe zugrunde, ein textiles Flachmaterial der genannten Art bereitzustellen, welches in einem Webprozess herstellbar ist und welches zur sicheren Aufnahme von elektrischen und/oder elektronischen Bauteilen geeignet ist. Hierunter werden unter anderem Mikrocontroller, Akkus, Batterien, Sensoren zur Erfassung von Körper- und Bewegungssignalen sowie Aktoren zur Einwirkung auf den Körper verstanden.

Vorzugsweise soll das textile Flachmaterial sowohl für die Langzeitverwendung als auch für eine Mehrfachverwendung geeignet sein. Insbesondere könnte das textile Flachmaterial auch zur Unterstützung und Überwachung der Wundbehandlung und/oder Wundversorgung eingesetzt werden, und zwar insbesondere als Wundauflage oder integriert in eine Wundauflage oder einen Wundverband im weit verstandenen Sinn.

Unter medizintechnischen Messzwecken werden insbesondere die Aufnahme von (bio-)physiologischen Signalen oder Potentialen, wie z.B. des elektrischen Widerstands der Haut, des Herzschlags, der Bewegung des Körpers, verstanden.

Unter medizintechnischen Steuerzwecken werden insbesondere die Steuerung des Körpers und/oder die Einwirkung auf den Körper verstanden, wie z.B. das Herbeiführen einer Bewegung des Körpers, die Elektrostimulation der Muskeln und/oder Nerven, im Rahmen der Diagnostik, der Therapie, der Rehabilitation und/oder des Self-Tracking.

Zur Lösung dieser Aufgabe wird erfindungsgemäß ein textiles Flachmaterial mit den Merkmalen des Anspruchs 1 vorgeschlagen.

Es wird also ein textiles Flachmaterial vorgeschlagen, das wie folgt ausgebildet ist. Das textile Flachmaterial umfasst eine Längsrichtung, eine quer zur Längsrichtung verlaufende Querrichtung, eine Erstreckungsebene, eine Dickenrichtung orthogonal zur Erstreckungsebene, wenigstens einen sich in der Längsrichtung erstreckenden Trägerabschnitt mit einer Trägerlage, wenigstens einen sich in der Längsrichtung erstreckenden und in der Längsrichtung an den Trägerabschnitt anschließenden Taschenabschnitt mit wenigstens einer Tasche zur Aufnahme der elektrischen und/oder elektronischen Bauteile, wobei die wenigstens eine Tasche eine körperzugewandte erste Taschenlage und eine körperabgewandte zweite Taschenlage umfasst, die zwischen sich einen Taschenraum begrenzen, wobei die wenigstens eine Tasche wenigstens eine in der Querrichtung zugängliche Taschenöffnung aufweist, wobei das textile Flachmaterial in der Längsrichtung angeordnete Kettfäden und in der Querrichtung angeordnete Schussfäden umfasst, welche die Trägerlage, die erste Taschenlage und die zweite Taschenlage bilden, wobei in der Dickenrichtung zwischen der ersten Taschenlage und der zweiten Taschenlage zumindest ein sich in der Längsrichtung erstreckender und wenigstens abschnittsweise flottierend angeordneter biegsamer elektrischer Leiter vorgesehen ist, der einen Flottierungsabstand von wenigstens 5 mm aufweist.

Die Herstellung des textilen Flachmaterials in einem Webprozess ermöglicht eine kontinuierliche Herstellung des textilen Flachmaterials und der darin oder daran vorgesehenen Tasche. Die Anordnung der Tasche in einem Taschenabschnitt des textilen Flachmaterials erreicht eine gezielte Positionierung der darin aufnehmbaren elektrischen und/oder elektronischen Bauteile. Die Tasche ist zweiseitig oder vorzugsweise einseitig zugänglich. Ferner ist im Taschenabschnitt durch das Vorsehen von zwischen den Taschenlagen flottierend angeordneten biegsamen elektrischen Leitern eine verbesserte und sicherere elektrische Verbindung zwischen dem elektrischen und/oder elektronischen Bauteil und dem elektrischen Leiter möglich. Aufgrund der flottierenden Anordnung des elektrischen Leiters kann im Gegensatz zur aus dem Stand der Technik bekannten nur punktuellen Verbindung zwischen dem Bauteil und dem elektrischen Leiter eine großflächigere Verbindung erzielt werden.

Ferner schützt die körperzugewandte erste Taschenlage die Haut vor der ungewollten Berührung des aufnehmbaren elektrischen und/oder elektronischen Bauteils. Die körperabgewandte zweite Taschenlage deckt das elektrische und/oder elektronische Bauteil zumindest teilweise ab und verhindert eine ungewollte Berührung und/oder Einwirkung von außen auf das in der Tasche aufgenommene elektrische und/oder elektronische Bauteil.

Vorteilhafterweise kann der flottierend angeordnete elektrische Leiter aus einem elektrisch leitfähigen Filament, einem elektrisch leitfähigen Faden oder einem elektrisch leitfähigen Kupferdraht gebildet sein. Der elektrische Leiter ist vorzugsweise webbar und entlang der Längserstreckung und/oder orthogonal zur Längserstreckung elastisch verformbar, insbesondere biegsam und/oder dehnbar, sein. Ferner ist es denkbar, dass der elektrische Leiter in der Längsrichtung mehrere parallel zueinander angeordnete Filamente, insbesondere ein Faserbündel, oder mehrere Kupferdrähte, insbesondere eine aus mehreren Kupferdrähten gebildete Kupferlitze, umfasst.

Wenn vorliegend von elektrischen Leitern die Rede ist, so bedeutet dies, dass ein solcher elektrischer Leiter ein elektrisch leitfähiges Material umfasst, insbesondere damit beschichtet ist, oder aus einem elektrisch leitfähigen Material besteht. Ein als elektrisch leitfähig geltender Leiter weist dabei einen elektrischen Widerstand von höchstens 2000 Ohm/m, vorzugsweise von höchstens 1000 Ohm/m, bevorzugt von höchstens 500 Ohm/m auf. Dies bedeutet, dass ein Leiterstück von 1 m Länge bei einer an sich üblichen Widerstandsmessung einen elektrischen Widerstand von höchstens 2000 Ohm bzw. höchstens 1000 Ohm bzw. höchstens 500 Ohm aufweist.

Grundsätzlich sind bei einem Gewebe die Kett- und die Schussfäden miteinander verwoben, wobei z.B. bei einer Leinwandbindung der Schussfaden jeweils abwechselnd über einen Kettfaden und unter einen Kettfaden sowie der Kettfaden jeweils abwechselnd über einen Schussfaden und unter einen Schussfaden geführt sind. Bei der flottierenden Anordnung des elektrischen Leiters ist im Vergleich zur Leinwandbindung der elektrische Leiter nicht abwechselnd über einen Schussfaden und unter einen Schussfaden angeordnet, sondern der elektrische Leiter verläuft über mehrere, vorzugsweise über eine Vielzahl von Schussfäden flottierend.

Ein Abstand zwischen zwei aufeinanderfolgenden Bindungspunkten wird im Nachfolgenden Flottierungsabstand genannt. Es hat sich als vorteilhaft erwiesen, wenn der elektrische Leiter im Taschenabschnitt einen Flottierungsabstand zwischen 5 mm und 100 mm, insbesondere zwischen 5 mm und 50 mm, vorzugsweise zwischen 5 mm und 30 mm, bevorzugt zwischen 10 mm und 20 mm aufweist.

Dadurch dass der Leiter in dem Taschenabschnitt flottierend verläuft, ist der elektrische Leiter in der Tasche leicht zugänglich, sodass der elektrische Leiter z.B. durch eine Schere getrennt, hinsichtlich einer Isolierung bzw. Beschichtung angepasst und/oder mit Steckern oder ähnlichem versehen werden kann.

Es ist auch denkbar, dass der elektrische Leiter im Taschenabschnitt vollständig flottierend angeordnet ist, also dort keinen Bindungspunkt zum Taschenabschnitt aufweist. Die Bindungspunkte können im Trägerabschnitt und/oder im Übergang zwischen Trägerabschnitt und Taschenabschnitt vorliegen. Die vollständig flottierende Anordnung des elektrischen Leiters im Trägerabschnitt weist eine hohe Flexibilität bei der Verbindung mit einem einzusetzenden Bauteil auf, da der elektrische Leiter aufgrund der fehlenden Anbindung im Taschenabschnitt beweglich oder verschieblich ist.

Bei einer alternierenden Ausgestaltung des textilen Flachmaterials schließt sich ein weiterer Trägerabschnitt an den Taschenabschnitt in der Längsrichtung gegenüber dem Trägerabschnitt an, wobei der Taschenabschnitt in der Längsrichtung zwischen den Trägerabschnitten angeordnet ist. Bei dieser Ausführungsform ergibt sich also bei dem textilen Flachmaterial in der Längsrichtung eine Anordnung von Trägerabschnitt - Taschenabschnitt - Trägerabschnitt.

Es kann sich auch als vorteilhaft erweisen, wenn sich ein weiterer Taschenabschnitt an den Trägerabschnitt in der Längsrichtung gegenüber dem Taschenabschnitt anschließt, wobei der Trägerabschnitt in der Längsrichtung zwischen den Taschenabschnitten angeordnet ist. Bei dieser weiteren Ausführungsform ergibt sich also bei dem textilen Flachmaterial in der Längsrichtung eine Anordnung von Taschenabschnitt - Trägerabschnitt - Taschenabschnitt.

Es kann sich als besonders vorteilhaft erweisen, wenn das textile Flachmaterial in der Längsrichtung im Wesentlichen endlos ausgebildet ist, wobei sich jeweils ein Trägerabschnitt und ein Taschenabschnitt abwechseln. Dadurch können insbesondere die Vorteile der kontinuierlichen Herstellung im Webprozess ausgeschöpft werden.

Hinsichtlich der Aufnahme der elektrischen und/oder elektronischen Bauteile erweist es sich als besonders vorteilhaft, wenn die erste Taschenlage und die zweite Taschenlage zumindest bereichsweise im Taschenabschnitt lose aneinander an- oder aufliegen. Folglich ist ein einfaches Einbringen des Bauteils in die Tasche möglich.

Hinsichtlich einer kontinuierlichen Herstellung ist es ferner vorteilhaft, wenn die sich in der Längsrichtung erstreckenden Kettfäden ausgehend vom Taschenabschnitt im Übergang zum Trägerabschnitt zur Bildung der Trägerlage zusammengeführt sind. Wenn das textile Flachmaterial in der Längsrichtung aufeinander folgend mit jeweils einem Taschenabschnitt und einem Trägerabschnitt ausgebildet ist, so alternieren mehrlagige Taschenabschnitte und einlagige Trägerabschnitte. Dies hat zum einen den Vorteil, dass mit den selben Kettfäden sowohl die Trägerlage als auch die Taschenlage gebildet werden kann, und zum anderen, dass im Trägerabschnitt eine stabile Trägerlage gebildet werden kann.

Ferner ist es vorteilhaft, wenn der wenigstens eine elektrische Leiter an der Trägerlage angeordnet ist, insbesondere teilweise von der Trägerlage umschlossen oder daran in sonstiger Weise gehalten ist. Dadurch wird der Leiter von den Kettfäden in einer Bewegung in der Querrichtung begrenzt. Zur weiteren Fixierung des Leiters erweist es sich als vorteilhaft, wenn der Leiter an der Trägerlage zumindest punktuell angebunden ist. Der elektrische Leiter kann hierfür in die Trägerlage textil eingebunden sein, oder der elektrische Leiter kann über Haltemittel an der Trägerlage fixiert sein.

In einer weiteren Fortbildung des erfindungsgemäßen Flachmaterials können mehrere in der Längsrichtung im Wesentlichen parallel zueinander verlaufende elektrische Leiter vorgesehen sein, wobei die elektrischen Leiter in der Querrichtung voneinander beabstandet sind, sodass bei einem bestimmungsgemäßen Gebrauch des textilen Flachmaterials kein unmittelbarer Kontakt zwischen zwei oder mehreren elektrischen Leitern besteht. Folglich können unterschiedliche Signale im und entlang des textilen Flachmaterials übertragen werden. Außerdem kann jeweils eine sichere Verbindung zwischen den Leitern und unterschiedlichen Kontaktstellen des aufgenommenen elektrischen und/oder elektronischen Bauteils gewährleistet werden.

Ferner ist es vorteilhaft, wenn der elektrische Leiter wenigstens abschnittsweise im Trägerabschnitt isoliert ist. So kann eine ungewollte Berührung oder Kontaktierung des elektrischen Leiters durch die anliegende Haut oder von außen verhindert werden.

Insbesondere kann es sich weiter als vorteilhaft erweisen, wenn der wenigstens eine elektrische Leiter im Taschenabschnitt wenigstens abschnittsweise nicht isoliert bzw. abisoliert ist, sodass ein elektrischer Kontakt zwischen dem wenigstens einen elektrischen Leiter und dem aufgenommenen elektrischen und/oder elektronischen Bauteil herstellbar ist. Der elektrische Leiter kann vor dem Einweben in das textile Flachmaterial mit freiliegenden, nicht isolierten Bereichen bereitgestellt werden. Alternativ kann der elektrische Leiter vollständig isoliert in das Flachmaterial eingebracht werden, und dann im Taschenabschnitt bedarfsgerecht abisoliert werden. Eine Kontaktierung der elektrischen Leiter und des aufgenommenen Bauteils erfolgt vorzugsweise auf der körperabgewandten Seite des Bauteils, sodass sich kein erhabenes Profil der elektrischen Leiter auf der körperzugewandten Seite ausbilden kann.

Eine Weiterbildung des erfindungsgemäßen Flachmaterials sieht vor, dass der wenigstens eine elektrische Leiter im Taschenabschnitt getrennt ist, sodass dieser elektrische Leiter zwei freie Leiterenden, insbesondere zur Kontaktierung des elektrischen und/oder elektronischen Bauteils aufweist. Aufgrund der Flottierung des elektrischen Leiters im Taschenabschnitt kann der Leiter nach dem Einbringen in das Flachmaterial, z.B. mit einer Schere, leicht getrennt werden.

Es ist nun möglich, die freien Leiterenden des getrennten elektrischen Leiters mit wenigstens einem Stecker zu versehen, sodass eine elektrische Kontaktierung mit dem aufgenommenen elektrischen und/oder elektronischen Bauteil über eine Steckverbindung herstellbar ist. Dazu sieht der Stecker und das Bauteil jeweils zueinander korrespondierende Anschlüsse vor. Folglich ist ein leichtes Anschließen und Entfernen des Bauteils durch Herstellen und Lösen der Steckverbindung möglich.

Nach einer weiteren Ausführungsform erweist es sich als vorteilhaft, dass die wenigstens eine Tasche durch Zusammenführen der ersten Taschenlage und der zweiten Taschenlage in der Querrichtung der Taschenöffnung gegenüberliegend geschlossen ist. Hinsichtlich des kontinuierlichen Herstellungsprozesses kann folglich ein textiles Flachmaterial mit Taschen gewebt werden, welche in der Querrichtung auf einer Seite eine Taschenöffnung aufweisen und auf der anderen Seite geschlossen sind.

An die wenigstens eine Tasche schließt in der Querrichtung gegenüber der Taschenöffnung ein Seitenbereich an, wobei in dem Seitenbereich die erste Taschenlage und die zweite Taschenlage flächenhaft miteinander verbunden sind, insbesondere in diesem Seitenbereich die in der Querrichtung verlaufenden Schussfäden der ersten Taschenlage und der zweiten Taschenlage und die in der Längsrichtung verlaufenden Kettfäden der ersten Taschenlage und der zweiten Taschenlage miteinander verwoben sind. Es ist zudem hinsichtlich der kontinuierlichen Herstellbarkeit des Flachmaterials denkbar, dass die Quererstreckung des Taschenabschnitts im Wesentlichen der Quererstreckung des Trägerabschnitts entspricht.

Ferner ist es vorteilhaft, wenn ein Positioniermittel zur Positionierung und/oder Arretierung eines in die Tasche einsetzbaren elektrischen und/oder elektronischen Bauteils an oder in der Tasche vorgesehen ist. Demnach kann das Bauteil sicher in der Tasche gehalten werden und vorzugsweise in einer definierten Position fixiert werden, sodass die Kontaktstellen des elektrischen und/oder elektronischen Bauteils und die elektrischen Leiter aneinander anliegen oder zueinander zugeordnet sind. Als Positioniermittel könnte insbesondere ein Haken am Bauteil und eine mit dem Haken korrespondierende Hinterschneidung im Taschenabschnitt vorgesehen sein. Es ist auch ein Magnet am Bauteil und ein ferromagnetisches Material im Taschenabschnitt denkbar.

Bei einer weiteren besonders vorteilhaften Ausführungsform ist vorgesehen, dass im Trägerabschnitt und/oder im Taschenabschnitt sich in der Längsrichtung erstreckende elastisch dehnbare Kettfäden und/oder sich in der Querrichtung erstreckende elastisch dehnbare Schussfäden vorgesehen sind. Folglich wird eine gute Anschmiegsamkeit des textilen Flachmaterials an den Körper gewährleistet. Ferner können die elastischen Fäden im Taschenabschnitt derart ausgebildet sein, dass ein eingebrachtes elektrisches und/oder elektronisches Bauteil haltend umschlossen wird, sodass ein Herausfallen des Bauteils aus der Tasche verhindert wird. Zudem können unterschiedliche elastische Fäden in der ersten Taschenlage und der zweiten Taschenlage vorgesehen sein. Insbesondere ist es denkbar, dass in der zweiten Taschenlage mehr elastische Fäden vorgesehen sind, als in der ersten Taschenlage. Dadurch kann gewährleistet werden, dass bei einem eingebrachten Bauteil die körperabgewandte zweite Taschenlage mehr als die körperzugewandte erste Taschenlage nachgibt, sodass das Bauteil von der Haut weggedrängt wird und somit ein geringerer Druck auf der Haut des Patienten lastet.

Die Elastizität der genannten elastischen Fäden kann analog nach DIN EN ISO 13934-1 bestimmt werden. Dabei werden anstelle der in der Norm genannten flächigen Probenstücke Fadenabschnitt einer Länge von je 80 mm verwendet. Anhand der Höchstzugkraftdehnung des verwendeten Fadens wird die Elastizität des Fadens bestimmt.

Dabei erweist es sich als vorteilhaft, wenn die Höchstzugkraftdehnung der elastischen Fäden wenigstens das 1,10-fache, insbesondere wenigstens das 2,0-fache und weiter insbesondere wenigstens das 5,0-fache, insbesondere wenigstens das 10-fache, insbesondere wenigstens das 20-fache der weiteren verwendeten Fäden beträgt. Weiter erweist es sich als vorteilhaft, wenn die Höchstzugkraftdehnung der elastischen Fäden in einem Bereich zwischen 10% und 300%, insbesondere in einem Bereich zwischen 10% und 200%, vorteilhafterweise in einem Bereich zwischen 10% und 100%, bevorzugt in einem Bereich zwischen 10% und 20% liegt.

Gegenstand der Erfindung ist auch eine Halte- oder Tragevorrichtung für ein elektrisches und/oder elektronisches Bauteil, welches ein erfindungsgemäßes textiles Flachmaterial umfasst. Das textile Flachmaterial ist dabei vorzugsweise als Halsband, Armband, Stirnband, Brustgürtel oder Kinesio-Tape vorkonfektioniert.

Es ist ferner denkbar, dass das Flachmaterial als Binde oder Bandage ausgebildet ist, wobei das endlos hergestellte Flachmaterial in einer definierten Länge geschnitten wird. Folglich bildet das Flachmaterial in der Längsrichtung Endbereiche mit freien Enden aus. Die Endbereiche des Flachmaterials können dann miteinander verbunden, insbesondere vernäht, verschweißt und/oder verklebt werden, sodass eine ringartige Binde oder Bandage entsteht. Alternativ können in dem Endbereich in der Längsrichtung Trägerabschnitte vorgesehen sein, wobei daran insbesondere Klettverschlüsse oder Klebemittel angebracht werden können. Mittels der Klettverschlüsse können Bandagen gezielt an das anliegende Körperteil angepasst werden. Mittels der Klebemittel kann das Flachmaterial als Pflaster eingesetzt werden.

Ferner wird Schutz beansprucht für eine Vorrichtung für medizintechnische Mess- und/oder Steuerzwecke zur Anwendung am menschlichen oder tierischen Körper, umfassend eine erfindungsgemäße Halte- oder Tragevorrichtung und ein daran gehaltenes und elektrisch kontaktiertes elektrisches und/oder elektronisches Bauteil, insbesondere ein Mikrocontroller, eine Batterie, ein Akku, ein Sensor zur Erfassung von Körper- und/oder Bewegungssignalen und/oder ein Bauteil zur Erzeugung und/oder Abgabe von elektrischen Signalen an den Körper. Es ist dabei denkbar, dass das Bauteil über eine Steckverbindung mit dem wenigstens einen elektrischen Leiter verbunden ist. Bei einem quaderförmigen Bauteil kann die Steckverbindung an den den Trägerabschnitten zugewandten Seiten, an den der Durchgangsöffnung zugewandten oder abgewandten Seite und/oder an den den Taschenlagen zugewandten Seiten des Bauteils vorgesehen sein.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführung des erfindungsgemäßen textilen Flachmaterials.

In der Zeichnung zeigt:
- Figur 1: eine schematische Draufsicht auf eine körperzugewandte Seite eines erfindungsgemäßen textilen Flachmaterials;
- Figur 2: eine schematische dreidimensionale Darstellung des textilen Flachmaterials gemäß Fig. 1 mit einem eingesetzten elektronischen Bauteil;
- Figur 3: eine schematische Seitenansicht des Flachmaterials gemäß Fig. 1 gesehen in Richtung des Pfeils III in Fig. 1;
- Figur 4: eine schematische Seitenansicht des Flachmaterials gemäß Fig. 1 und 2 mit einem in eine Tasche eingesetzten elektronischen Bauteil;
- Figur 5a: eine schematische Darstellung eines als Binde ausgebildeten erfindungsgemäßen textilen Flachmaterials mit Haltemitteln; und
- Figur 5b: eine schematische Darstellung einer Binde mit einem darin aufgenommenen elektronischen Bauteil in einer Betriebssituation an einem Oberarm einer Person.

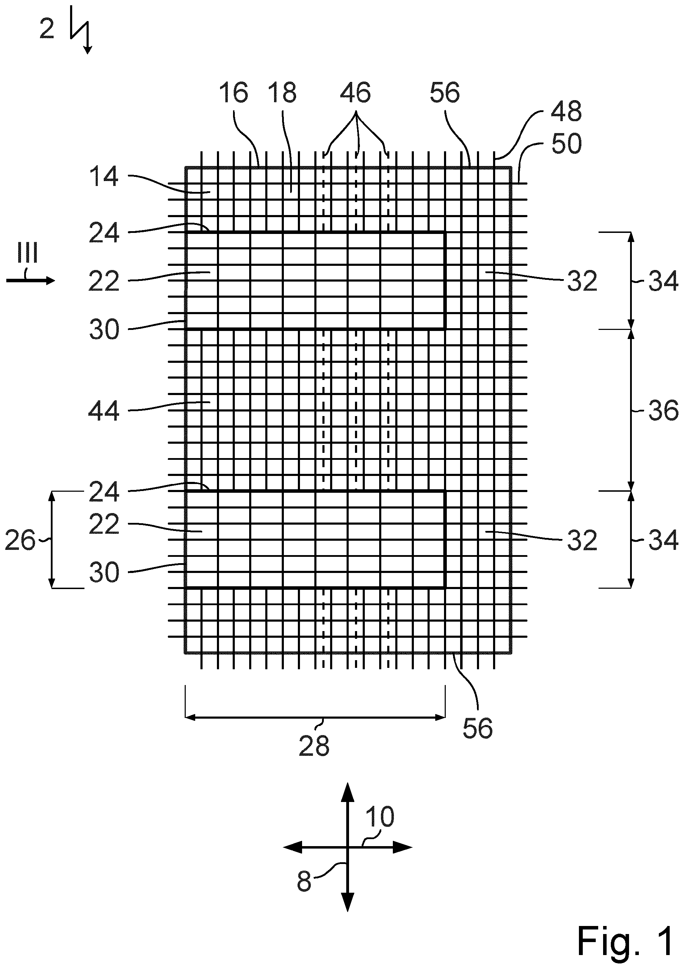

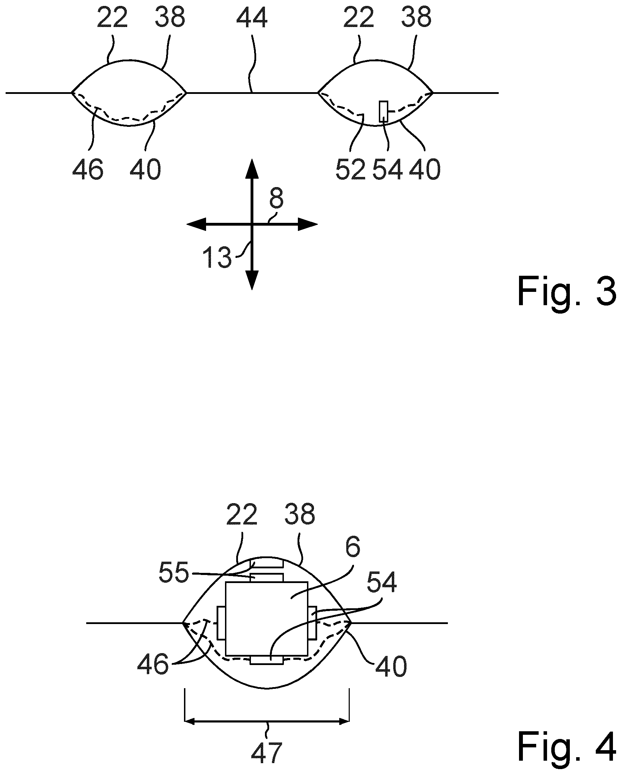

Die Figuren zeigen ein erfindungsgemäßes textiles Flachmaterial 2 und verdeutlichen teils stark schematisch dessen Aufbau. Das Flachmaterial 2 ist dazu vorgesehen, an einen menschlichen oder tierischen Körper 4 angelegt zu werden und wenigstens ein elektrisches und/oder elektronisches Bauteil 6 aufzunehmen. Das textile Flachmaterial 2 erstreckt sich in einer Längsrichtung 8, in einer Querrichtung 10 und in einer mit der Zeichnungsebene der Fig. 1 übereinstimmenden Erstreckungsebene 12. Ferner ist eine orthogonal zur Erstreckungsebene 12 verlaufende Dickenrichtung 13 eingeführt, die aus Fig. 3 ersichtlich ist.

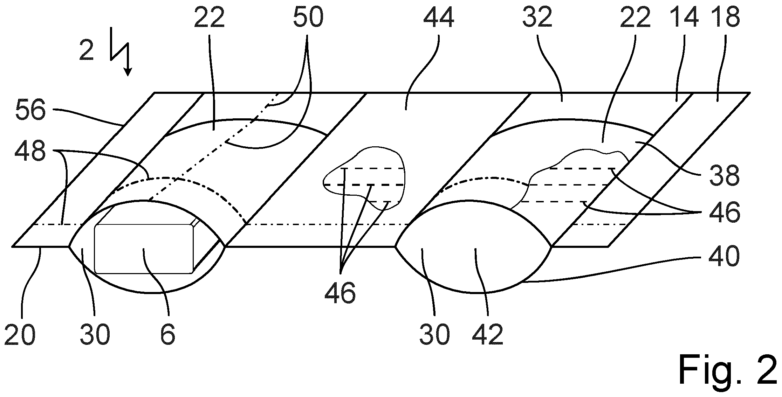

Fig. 1 zeigt eine Draufsicht auf eine körperzugewandte Seite 14 eines durch einen rechteckförmigen Rahmen 16 angedeuteten Abschnitts 18 des textilen Flachmaterials 2. Fig. 2 zeigt eine dreidimensionale Darstellung des textilen Flachmaterials 2, wobei die körperzugewandte Seite 14 nach oben und die körperabgewandte Seite 20 - nicht sichtbar - nach unten orientiert ist.

Der Abschnitt 18 des textilen Flachmaterials 2 umfasst zur Aufnahme von elektrischen und/oder elektronischen Bauteilen 6 zwei im hier dargestellten Fall beispielhaft rechteckig ausgebildete Taschen 22, die in Fig. 1 mit einem Rahmen 24 angedeutet sind. Die Taschen 22 erstrecken sich in der Längsrichtung 8 über eine Taschenlänge 26 und in der Querrichtung 10 über eine Taschenbreite 28. Die Taschen 22 sind in der Querrichtung 10 über eine an einem seitlichen Rand des Abschnitts 18 vorgesehene Taschenöffnung 30 zugänglich. In der Querrichtung 10 gegenüber der Taschenöffnung 30 schließt sich an die Tasche 22 ein Seitenbereich 32 des Abschnitts 18 an. Der Seitenbereich 32 erstreckt sich in der Längsrichtung 8 über die Taschenlänge 26 und in der Querrichtung 10 zwischen der Tasche 22 und einem seitlichen Rand des Abschnitts 18.

Der Bereich der jeweiligen Tasche 22 bildet zusammen mit dem angrenzenden Seitenbereich 32 jeweils einen Taschenabschnitt 34 des textilen Flachmaterials 2 bzw. des Abschnitts 18. In der Längsrichtung 8 zwischen den beiden Taschenabschnitten 34 ist ein Trägerabschnitt 36 vorgesehen bzw. angeordnet.

Wie nachfolgend noch im Einzelnen erläutert wird, kann das erfindungsgemäße textile Flachmaterial 2 in der Längsrichtung 8 endlos herstellt werden, sodass aus einem endlos hergestellten Streifen oder Band des erfindungsgemäßen textilen Flachmaterials 2 die in den Figuren dargestellten Abschnitte 18 des Flachmaterials 2 als Längsabschnitte dieses endlosen Flachmaterials 2 erhalten werden können. So ist es denkbar, dass ein Abschnitt 18 eine oder mehrere Taschen 22 aufweisen kann, zwischen denen in der Längsrichtung 8 - wie dargestellt - jeweils ein Trägerabschnitt 36 angeordnet ist.

In Fig. 2 ist der Abschnitt 18 des textilen Flachmaterials 2 ebenfalls ersichtlich, wobei erkennbar ist, dass das textile Flachmaterial 2 im jeweiligen Taschenabschnitt 34 eine körperzugewandte erste Taschenlage 38 und eine körperabgewandte zweite Taschenlage 40 umfasst. Die körperzugewandte erste Taschenlage 38 und die körperabgewandte zweite Taschenlage 40 sind im bevorzugt dargestellten Fall insbesondere lose gegeneinander anlegbar und/oder derart aufweitbar, dass ein Taschenraum 42 von der ersten Taschenlage 38 und der zweiten Taschenlage 40 begrenzt wird. In diesem Taschenraum 42 kann dann das elektrische und/oder elektronische Bauteil 6 aufgenommen werden.

Anhand der Fig. 2 bis 4 ist ersichtlich, dass die körperzugewandte erste Taschenlage 38 und die körperabgewandte zweite Taschenlage 40 zur Bildung einer im Trägerabschnitt 36 vorgesehenen textilen Trägerlage 44 zusammengeführt sind. Die Trägerlage 44 erstreckt sich in der Querrichtung 10 zwischen den seitlichen Rändern des Abschnitts 18 des Flachmaterials 2, also über dessen gesamte Breite. In der Längsrichtung 8 erstreckt sich die Trägerlage 44 zwischen den beiden Taschen 22.

Vorzugsweise erfüllt das textile Flachmaterial 2 neben der Funktion der Aufnahme des elektrischen und/oder elektronischen Bauteils 6 die Funktion der elektrischen Verbindung des aufgenommenen Bauteils 6 mit weiteren elektrischen und/oder elektronischen Bauteilen, die außerhalb dieser Tasche 22 liegen. Diese können z.B. in weiteren Taschen 22 des selben textilen Flachmaterials 2 angeordnet sein oder z.B. als eine Elektrode an einem weiteren Funktionsabschnitt des textilen Flachmaterials 2 in Form eines Elektrodenabschnitts ausgebildet und angeordnet sein.

Zur Verbindung bzw. Kontaktierung des elektrischen und/oder elektronischen Bauteils 6 sind in dieser Ausführungsform beispielhaft drei elektrische Leiter 46 vorgesehen, welche in das textile Flachmaterial 2 eingebracht sind. In den Fig. 1 bis 5 sind die elektrischen Leiter 46 als gestrichelte Linien dargestellt. Die elektrischen Leiter 46 dienen zur Übertragung von Signalen vom Körper weg, zum Körper hin und/oder zwischen zwei elektrischen und/oder elektronischen Bauteilen 6, wobei jeder elektrische Leiter 46 ein unterschiedliches Signal übertragen kann. Die elektrischen Leiter 46 sind in der Längsrichtung 8 und parallel zueinander verlaufend angeordnet. Im Trägerabschnitt 36 sind die elektrischen Leiter 46 derart mit der Trägerlage 44 verbunden, dass ein ungewolltes Verrutschen der elektrischen Leiter 46 in der Längsrichtung 8 und/oder in der Querrichtung 10 verhindert wird. Ferner sind die elektrischen Leiter 46 derart in die Trägerlage 44 eingebunden, dass ein Berühren der elektrischen Leiter 46 untereinander bei einem sachgemäßen Gebrauch verhindert werden kann. Zudem sind die elektrischen Leiter 46 im Taschenabschnitt 34 nicht an der ersten Taschenlage 38 und nicht an der zweiten Taschenlage 40 angebunden. Folglich liegen die elektrischen Leiter 46 in der Tasche 22 weitestgehend frei zwischen der ersten Taschenlage 38 und der zweiten Taschenlage 40, wobei sie lose an die erste Taschenlage 38 und/oder an die zweite Taschenlage 40 von innen anliegen können.

Nachfolgend wird die Bildung der Lagen, der Taschen, der Bereiche und der Abschnitte des erfindungsgemäßen textilen Flachmaterials erläutert.

Die erste Taschenlage 38, die zweite Taschenlage 40 und die Trägerlage 44 umfassen in der Längsrichtung 8 erstreckte Kettfäden 48 und in der Querrichtung 10 erstreckte Schussfäden 50, welche insbesondere als elektrisch nicht leitfähige Fäden ausgebildet sind.

Der Verlauf eines einzigen Kettfadens 48 und eines einzigen Schussfadens 50 ist in Fig. 2 schematisch angedeutet. Der Kettfaden 48 und der Schussfaden 50 werden zur Bildung der zweiten Taschenlage 40 im Taschenabschnitt 34 aus der Nullstellung abgesenkt und miteinander verwoben. Analog werden zur Bildung der ersten Taschenlage 38 weitere Kettfäden 48 und Schussfäden 50 aus der Nullstellung angehoben und miteinander verwoben. Im Übergang zum Taschenabschnitt werden die Kettfäden 48 und die Schussfäden 50 in die Nullstellung zurück angehoben bzw. abgesenkt. Folglich werden die in der Längsrichtung 8 sich erstreckenden Kettfäden 48, welche die erste Taschenlage 38 bilden, und die in der Längsrichtung 8 sich erstreckenden Kettfäden 48, welche die zweite Taschenlage 40 bilden, zur Bildung der gewebten Trägerlage 44 im Trägerabschnitt 36 zusammengeführt. Entsprechend bildet sich eine stabile Trägerlage 44 aus den Kettfäden 48 und den Schussfäden 50 in der Nullstellung.

Entsprechend erfolgt die Bildung des Seitenbereichs 32. Die Schussfäden 50, welche jeweils die erste Taschenlage 38 und die zweite Taschenlage 40 bilden, sind im Bereich der Tasche 22 angehoben bzw. abgesenkt. Im Übergang von der Tasche 22 zum Seitenbereich 32 werden die jeweiligen Schussfäden 50 zurück in die Nullstellung abgesenkt bzw. angehoben und mit weiteren Kettfäden 48 verwoben, sodass eine einzige textile Lage im Seitenbereich 32 gebildet werden kann, welche mit der Trägerlage 44 vergleichbar ist. Es könnte dort aber alternativ auch nachträglich die beiden Taschenlagen 38, 40 auf an sich beliebige Weise untereinander verbunden werden.

Insbesondere aus Fig. 2 und 3 ist der Verlauf der elektrischen Leiter 46 ersichtlich. Die elektrischen Leiter 46 sind im Trägerabschnitt 36 nahezu vollständig von den Kettfäden 48 und den Schussfäden 50 im Trägerabschnitt 36 abgedeckt. Im Taschenabschnitt 34 sind die elektrischen Leiter 46 beispielhaft vollständig flottierend angeordnet. Folglich sind die elektrischen Leiter 46 im Taschenabschnitt 34 nicht an die erste Taschenlage 38 und nicht an die zweite Taschenlage 40 angebunden. Die elektrischen Leiter 46 sind im Rahmen ihrer Spielbehaftung und Elastizität frei in der Tasche 22 beweglich, wobei die elektrischen Leiter 46 jeweils zwei Bindungspunkte im Übergang zwischen dem Taschenabschnitt 34 und dem Trägerabschnitt 36 aufweisen. Folglich entspricht der Flottierungsabstand 47 der elektrischen Leiter 46 im Taschenabschnitt 34 ungefähr der Taschenlänge 26. Es ist beispielhaft denkbar, dass die elektrischen Leiter 46 an der ersten Taschenlage 38 und/oder an der zweiten Taschenlage 40 mit einem Flottierungsabstand 47 zwischen 5 mm und 30 mm flottierend angeordnet sind. Bei einer beispielhaften Taschenlänge 26 von 30 mm und einem Flottierungsabstand 47 von 5 mm sind die elektrischen Leiter 46 in den Übergängen zwischen dem Taschenabschnitt 34 und dem Trägerabschnitt 36 sowie an fünf weiteren Bindungspunkten angebunden. In diesem Fall bilden sich im Taschenabschnitt 34 sechs flottierende Bereiche der elektrischen Leiter 46 aus. Es ist ferner möglich, dass bei mehreren elektrischen Leitern 46 die einzelnen elektrischen Leiter 46 unterschiedlich im Taschenabschnitt 34 und/oder im Trägerabschnitt 36 angeordnet sind, insbesondere im Taschenabschnitt 34 unterschiedlich flottierend angeordnet sind. Die elektrischen Leiter 46 können im Taschenabschnitt jeweils einen Flottierungsabstand vorzugsweise zwischen 5 mm und 100 mm, insbesondere zwischen 5 mm und 50 mm, vorzugsweise zwischen 5 mm und 30 mm, bevorzugt zwischen 10 mm und 20 mm aufweisen.

Die flottierende Anordnung der elektrischen Leiter 46 sind in Fig. 3 als wellenförmige gestrichelte Linie angedeutet. In der linken Tasche 22 ist der elektrische Leiter 46 durchgehend, wobei z.B. ein elektrisches und/oder elektronisches Bauteil 6 mit offenen Kontaktstellen in die Tasche 22 eingesetzt werden kann, sodass durch die Ausrichtung des Bauteils 6 in der Tasche 22 eine anliegende Verbindung zwischen dem Bauteil 6 und den elektrischen Leitern 46 hergestellt werden kann. Demnach ist es sinnvoll, dass die elektrischen Leiter 46 im Taschenabschnitt 34 elektrisch kontaktierbar sind. Die elektrischen Leiter 46 können mit einer Isolierung in das textile Flachmaterial 2 eingebracht werden. Im Taschenabschnitt 34 müssen dann die elektrischen Leiter 46 abisoliert werden. Es ist ferner denkbar, dass die elektrischen Leiter 46 abschnittsweise keine Isolierung aufweisen und beim Einbringen der elektrischen Leiter 46 in das Flachmaterial 2 die nicht isolierten Bereiche an die Taschenabschnitte 34 ausgerichtet werden. In der rechten Tasche 22 ist der elektrische Leiter 46 mittels einer Schere getrennt worden. Folglich weist der elektrische Leiter 46 zwei freie Leiterenden 52 auf. In Abhängigkeit des Leitertyps kann das freie Leiterende 52 mit einem einzusetzenden Bauteil 6 verbunden werden. Das linke freie Leiterende 52 kann z.B. an eine Kontaktstelle eines einzusetzenden Bauteils 6 angefügt, insbesondere geklebt oder gelötet werden. An das rechte freie Leiterende 52 ist ein Stecker 54 vorgesehen, mit welchem eine Steckverbindung zu einem einzusetzenden Bauteil 6 hergestellt werden kann. Das elektrische und/oder elektronische Bauteil 6 weist entsprechend einen zum Stecker 54 korrespondierenden Steckplatz auf.

In Fig. 4 ist eine Tasche 22 eines erfindungsgemäßen Flachmaterials 2 gezeigt. In der Tasche 22 ist ein elektronisches Bauteil 6 eingesetzt, welches vier Steckplätze für Stecker 54 umfasst. Das Flachmaterial 2 umfasst in diesem Fall zwei elektrische Leiter 46, welche an ihren freien Leiterenden 52 mit Steckern 54 ausgestattet sind. Die Stecker 54 sind in der Längsrichtung 8 bzw. in der Dickenrichtung 13 in das elektronische Bauteil 6 eingesteckt. Zur vereinfachten und besseren Ausrichtung des elektrischen und/oder elektronischen Bauteils sind Positioniermittel 55 am Bauteil 6 und der Tasche 22 vorgesehen, die beim Einsetzen des Bauteils 6 derart zusammenwirken, dass das Bauteil 6 und die Tasche 22 zusammenrasten und/oder der Benutzer einen Widerstand spürt. Als Positioniermittel 55 sind z.B. Magnete, Rasthaken mit Hinterschneidungen und/oder Kleber denkbar.

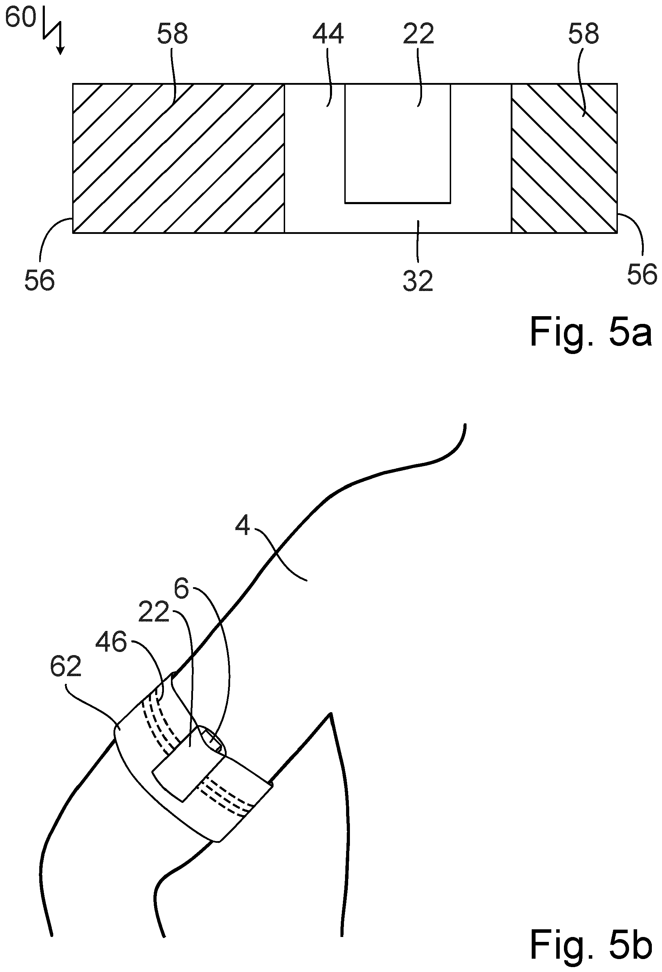

Die Fig. 5a und 5b zeigen beispielhaft das erfindungsgemäße textile Flachmaterial 2, welches als Binde 60 zur Anordnung an einem Arm konfektioniert ist. Die Binde 60 umfasst eine Tasche 22 und zwei Trägerlagen 44, welche in der Längsrichtung 8 an die Tasche 22 angrenzen. Die Binde 60 und das in der Tasche 22 aufgenommene elektronische Bauteil 6 bilden eine Vorrichtung 62 für medizintechnische Mess- und/oder Steuerzwecke. Im Fall der Binde 60 enden die Trägerlagen 44 in der Längsrichtung 8 in jeweils ein freies Trägerende 56. An den freien Trägerenden 56 der Trägerlagen 44 können jeweils Haltemittel 58 zur Herstellung der Haftung bzw. Fixierung am Arm vorgesehen sind. Zum Beispiel können diese Haltemittel 58 als Klebestellen ausgebildet sein, sodass das Flachmaterial 2 als Pflaster auf die Haut geklebt werden kann. In Fig. 5b ist alternativ eine Binde 60 gezeigt, welche einen Klettverschluss an den freien Trägerenden 56 der Trägerlagen 44 aufweist. Folglich können die freien Trägerenden 56 übereinandergelegt werden, sodass das Flachmaterial 2 individuell am Arm fixiert werden kann. Es ist ferner denkbar, dass die freien Trägerenden 56 miteinander vernäht werden, sodass eine Binde 60 mit einem definierten Umfang aus dem Flachmaterial 2 hergestellt werden kann. In diesem Fall ist es besonders vorteilhaft, wenn das Flachmaterial 2 elastische Fäden 48, 50 aufweist, sodass eine elastisch dehnbare, an die Haut anschmiegsame Binde 60 hergestellt werden kann. Die elastischen Fäden 48, 50 können auch in den anderen Ausführungsformen vorteilhaft Verwendung finden.

So können die elastischen Fäden 48, 50 auch vorteilhaft sein, wenn diese im Bereich der Taschenlagen 38, 40 vorgesehen sind, sodass ein darin aufgenommenes Bauteil 6 durch eine oder beide Taschenlagen 38, 40 umschlossen werden und so nicht aus der Tasche 22 fallen kann.

## Patentansprüche

1. Textiles Flachmaterial (2) zum Anlegen an einen menschlichen oder tierischen Körper (4) und zur Aufnahme von elektrischen und/oder elektronischen Bauteilen (6) für medizintechnische Mess- und/oder Steuerzwecke, umfassend:
eine Längsrichtung (8),
eine quer zur Längsrichtung (8) verlaufende Querrichtung (10),
eine Erstreckungsebene (12),
eine Dickenrichtung (13) orthogonal zur Erstreckungsebene (12),
wenigstens einen sich in der Längsrichtung (8) erstreckenden Trägerabschnitt (36) mit einer Trägerlage (44),
wenigstens einen sich in der Längsrichtung (8) erstreckenden und in der Längsrichtung (8) an den Trägerabschnitt (36) anschließenden Taschenabschnitt (34) mit wenigstens einer Tasche (22) zur Aufnahme der elektrischen und/oder elektronischen Bauteile (6),
wobei die wenigstens eine Tasche (22) eine körperzugewandte erste Taschenlage (38) und eine körperabgewandte zweite Taschenlage (40) umfasst, die zwischen sich einen Taschenraum (42) begrenzen,
wobei die wenigstens eine Tasche (22) wenigstens eine in der Querrichtung (10) zugängliche Taschenöffnung (30) aufweist,
wobei das textile Flachmaterial (2) in der Längsrichtung (8) angeordnete Kettfäden (48) und in der Querrichtung (10) angeordnete Schussfäden (50) umfasst, welche die Trägerlage (44), die erste Taschenlage (38) und die zweite Taschenlage (40) bilden,
wobei in der Dickenrichtung (13) zwischen der ersten Taschenlage (38) und der zweiten Taschenlage (40) zumindest ein sich in der Längsrichtung (8) erstreckender und wenigstens abschnittsweise flottierend angeordneter biegsamer elektrischer Leiter (46) vorgesehen ist und wobei der flottierend angeordnete elektrische Leiter (46) einen Flottierungsabstand (47) von wenigstens 5 mm aufweist.

2. Textiles Flachmaterial (2) nach Anspruch 1, **dadurch gekennzeichnet, dass** der flottierend angeordnete elektrische Leiter (46) einen Flottierungsabstand (47) von wenigstens 10 mm, und/oder von höchstens 100 mm, insbesondere höchstens 50 mm, insbesondere höchstens 30 mm, insbesondere höchstens 20 mm aufweist.

3. Textiles Flachmaterial (2) nach Anspruch 1 oder 2, **dadurch gekennzeichnet, dass** ein weiterer Trägerabschnitt (36) an den Taschenabschnitt (34) in der Längsrichtung (8) gegenüber dem Trägerabschnitt (36) anschließt, wobei der Taschenabschnitt (34) in der Längsrichtung (8) zwischen den Trägerabschnitten (36) angeordnet ist und/oder dass ein weiterer Taschenabschnitt (34) an den Trägerabschnitt (36) in der Längsrichtung (8) gegenüber dem Taschenabschnitt (34) anschließt, wobei der Trägerabschnitt (36) in der Längsrichtung (8) zwischen den Taschenabschnitten (34) angeordnet ist.

4. Textiles Flachmaterial (2) nach einem der vorhergehenden Ansprüche, **dadurch gekennzeichnet, dass** das textile Flachmaterial (2) in der Längsrichtung (8) endlos ausgebildet ist, wobei sich jeweils ein Trägerabschnitt (36) und ein Taschenabschnitt (34) abwechseln.

5. Textiles Flachmaterial (2) nach einem der vorhergehenden Ansprüche, **dadurch gekennzeichnet, dass** die sich in der Längsrichtung (8) erstreckenden Kettfäden (48) ausgehend vom Taschenabschnitt (34) im Übergang zum Trägerabschnitt (36) zur Bildung der Trägerlage (44) zusammengeführt sind.

6. Textiles Flachmaterial (2) nach einem der vorhergehenden Ansprüche, **dadurch gekennzeichnet, dass** der wenigstens eine elektrische Leiter (46) an der Trägerlage (44) angeordnet ist, insbesondere teilweise von der Trägerlage (44) umschlossen oder daran in sonstiger Weise gehalten ist.

7. Textiles Flachmaterial (2) nach einem der vorhergehenden Ansprüche, **dadurch gekennzeichnet, dass** mehrere in der Längsrichtung (8) im Wesentlichen parallel zueinander verlaufenden elektrischen Leiter (46) vorgesehen sind, wobei die elektrischen Leiter (46) in der Querrichtung (10) voneinander derart beabstandet sind, dass bei einem bestimmungsgemäßen Gebrauch des textilen Flachmaterials (2) kein unmittelbarer Kontakt zwischen zwei oder mehreren elektrischen Leitern (46) besteht.

8. Textiles Flachmaterial (2) nach einem der vorhergehenden Ansprüche, **dadurch gekennzeichnet, dass** der wenigstens eine elektrische Leiter (46) im Trägerabschnitt (36) wenigstens abschnittsweise isoliert ist und/oder dass der wenigstens eine elektrische Leiter (46) im Taschenabschnitt (34) wenigstens abschnittsweise nicht isoliert bzw. abisoliert ist, sodass ein elektrischer Kontakt zwischen dem wenigstens einen elektrischen Leiter (46) und dem elektrischen und/oder elektronischen Bauteil (6) herstellbar ist.

9. Textiles Flachmaterial (2) nach einem der vorhergehenden Ansprüche, **dadurch gekennzeichnet, dass** der wenigstens eine elektrische Leiter (46) im Taschenabschnitt (34) getrennt ist, sodass dieser elektrische Leiter (46) zwei freie Leiterenden (52) insbesondere zur Kontaktierung des elektrischen und/oder elektronischen Bauteils (6) aufweist.

10. Textiles Flachmaterial (2) nach Anspruch 9, **dadurch gekennzeichnet, dass** an wenigstens einem freien Leiterende (52) des getrennten elektrischen Leiters (46) ein Stecker (54) vorgesehen ist, sodass mit dem elektrischen und/oder elektronischen Bauteil (6) eine Steckverbindung herstellbar ist.

11. Textiles Flachmaterial (2) nach einem der vorhergehenden Ansprüche, **dadurch gekennzeichnet, dass** die wenigstens eine Tasche (22) durch Zusammenführen der ersten Taschenlage (38) und der zweiten Taschenlage (40) in der Querrichtung (10) der Taschenöffnung (30) gegenüberliegend geschlossen ist.

12. Textiles Flachmaterial (2) nach einem der vorhergehenden Ansprüche, **dadurch gekennzeichnet, dass** an die wenigstens eine Tasche (22) in der Querrichtung (10) gegenüber der Taschenöffnung (30) ein Seitenbereich (32) anschließt, wobei in dem Seitenbereich (32) die erste Taschenlage (38) und die zweite Taschenlage (40) flächenhaft miteinander verbunden sind, insbesondere in diesem Seitenbereich (32) die in der Querrichtung (10) verlaufenden Schussfäden (50) und die in der Längsrichtung (8) verlaufenden Kettfäden (48) miteinander verwoben sind.

13. Textiles Flachmaterial (2) nach einem der vorhergehenden Ansprüche, **dadurch gekennzeichnet, dass** ein Positioniermittel (55) zur Positionierung und/oder Arretierung eines in die Tasche (22) einsetzbaren elektrischen und/oder elektronischen Bauteils (6) an oder in der Tasche (22) vorgesehen ist.

14. Textiles Flachmaterial (2) nach einem der vorhergehenden Ansprüche, **dadurch gekennzeichnet, dass** im Trägerabschnitt (36) und/oder im Taschenabschnitt (34) sich in der Längsrichtung (8) erstreckende elastisch dehnbare Kettfäden (48) und/oder sich in der Querrichtung (10) erstreckende elastisch dehnbare Schussfäden (50) vorgesehen sind.

15. Halte- oder Tragevorrichtung (60) für ein elektrisches und/oder elektronisches Bauteil (6), umfassend:
ein textiles Flachmaterial (2) nach einem oder mehreren der vorhergehenden Ansprüche.

16. Vorrichtung (62) für medizintechnische Mess- und/oder Steuerzwecke zur Anwendung am menschlichen oder tierischen Körper (4), umfassend eine Halte- oder Tragevorrichtung (60) nach Anspruch 15 und ein daran gehaltenes und elektrisch kontaktiertes elektrisches und/oder elektronisches Bauteil (6), insbesondere ein Mikrocontroller, eine Batterie, ein Akku und/oder ein Sensor zur Erfassung von Körper- und/oder Bewegungssignalen oder ein Bauteil zur Erzeugung und/oder Abgabe von elektrischen Signalen an den Körper (4).

## Claims

1. A textile flat material (2) for application to a human or animal body (4) and for accommodating electrical and/or electronic components (6) for medical measuring and/or control purposes, comprising:
a longitudinal direction (8),
a transverse direction (10) running transversely to the longitudinal direction (8),
an extension plane (12),
a thickness direction (13) orthogonal to the extension plane (12),
at least one carrier portion (36) extending in the longitudinal direction (8) with a carrier layer (44),
at least one pocket portion (34) extending in the longitudinal direction (8) and adjoining the carrier portion (36) in the longitudinal direction (8) with at least one pocket (22) for accommodating the electrical and/or electronic components (6),
wherein the at least one pocket (22) comprises a first pocket layer (38) facing the body and a second pocket layer (40) facing away from the body, which define a pocket space (42) between them, wherein the at least one pocket (22) has at least one pocket opening (30) accessible in the transverse direction (10),
wherein the textile flat material (2) comprises warp threads (48) arranged in the longitudinal direction (8) and weft threads (50) arranged in the transverse direction (10), which form the carrier layer (44), the first pocket layer (38) and the second pocket layer (40),
wherein, in the thickness direction (13) between the first pocket layer (38) and the second pocket layer (40), at least one flexible electrical conductor (46) extending in the longitudinal direction (8) and arranged at in a floating manner at least in portions is provided, and wherein the electrical conductor (46) arranged in a floating manner has a floating distance (47) of at least 5 mm.

2. The textile flat material (2) according to claim 1, **characterized in that** the floating electrical conductor (46) has a floating distance (47) of at least 10 mm, and/or of at most 100 mm, in particular at most 50 mm, in particular at most 30 mm, in particular at most 20 mm.

3. The textile flat material (2) according to claim 1 or 2, **characterized in that** a further carrier portion (36) adjoins the pocket portion (34) in the longitudinal direction (8) opposite the carrier portion (36), wherein the pocket portion (34) is arranged in the longitudinal direction (8) between the carrier portions (36) and/or that a further pocket portion (34) adjoins the carrier portion (36) in the longitudinal direction (8) opposite the pocket portion (34), wherein the carrier portion (36) is arranged in the longitudinal direction (8) between the pocket portions (34).

4. The textile flat material (2) according to one of the preceding claims, **characterized in that** the textile flat material (2) is designed to be endless in the longitudinal direction (8), wherein a carrier portion (36) and a pocket portion (34) alternate with one another in each case.

5. The textile flat material (2) according to one of the preceding claims, **characterized in that** the warp threads (48) extending in the longitudinal direction (8) are brought together starting from the pocket portion (34) in the transition to the carrier portion (36) for forming the carrier layer (44).

6. The textile flat material (2) according to one of the preceding claims, **characterized in that** the at least one electrical conductor (46) is arranged on the carrier layer (44), in particular partially enclosed by the carrier layer (44) or held thereon in some other manner.

7. The textile flat material (2) according to one of the preceding claims, **characterized in** multiple electrical conductors (46) running substantially parallel to one another in the longitudinal direction (8) are provided, wherein the electrical conductors (46) are spaced apart from one another in the transverse direction (10) in such a way there is no direct contact between two or more electrical conductors (46) when the textile flat material (2) is used as intended.

8. The textile flat material (2) according to one of the preceding claims, **characterized in that** the at least one electrical conductor (46) in the carrier portion (36) is insulated at least in portions and/or that the at least one electrical conductor (46) in the pocket portion (34) is uninsulated or stripped at least in portions, so that an electrical contact can be established between the at least one electrical conductor (46) and the electrical and/or electronic component (6).

9. The textile flat material (2) according to one of the preceding claims, **characterized in that** the at least one electrical conductor (46) is separated in the pocket portion (34), so that this electrical conductor (46) has two free conductor ends (52), in particular for contacting the electrical and/or electronic component (6).

10. The textile flat material (2) according to claim 9, **characterized in that** a plug (54) is provided on at least one free conductor end (52) of the separate electrical conductor (46), so that a plug connection can be established with the electrical and/or electronic component (6).

11. The textile flat material (2) according to one of the preceding claims, **characterized in that** the at least one pocket (22) is closed by bringing together the first pocket layer (38) and the second pocket layer (40) in the transverse direction (10) opposite the pocket opening (30).

12. The textile flat material (2) according to one of the preceding claims, **characterized in that** a side region (32) adjoins the at least one pocket (22) in the transverse direction (10) opposite the pocket opening (30), wherein, in the side region (32), the first pocket layer (38) and the second pocket layer (40) are connected to one another in a flat manner, in particular in this side region (32) the weft threads (50) running in the transverse direction (10) and the warp threads (48) running in the longitudinal direction (8) are interwoven with one another.

13. The textile flat material (2) according to one of the preceding claims, **characterized in that** a positioning means (55) for positioning and/or locking an electrical and/or electronic component (6) that can be inserted into the pocket (22) is provided on or in the pocket (22).

14. The textile flat material (2) according to one of the preceding claims, **characterized in that**, in the carrier portion (36) and/or in the pocket portion (34), elastically stretchable warp threads (48) extending in the longitudinal direction (8) and/or elastically stretchable weft threads (50) extending in the transverse direction (10) are provided.

15. A holding or carrying device (60) for an electrical and/or electronic component (6), comprising:
a textile flat material (2) according to one or more of the preceding claims.

16. A device (62) for medical measurement and/or control purposes for use on the human or animal body (4), comprising a holding or carrying apparatus (60) according to claim 15 and an electrical and/or electronic component (6) held thereon and electrically contacted, in particular a microcontroller, a battery, a rechargeable battery and/or a sensor for detecting body and/or movement signals or a component for generating and/or emitting electrical signals to the body (4).

## Revendications

1. Matériau plat textile (2) destiné à être appliqué sur un corps humain ou animal (4) et destiné à recevoir des composants électriques et/ou électroniques (6) à des fins de mesure et/ou de commande de technique médical, comprenant:
une direction longitudinale (4),
une direction transversale (10) s'étendant transversalement à la direction longitudinale (8),
un plan d'extension (12),
une direction d'épaisseur (13) orthogonale au plan d'extension (12),
au moins une section de support (36) s'étendant dans la direction longitudinale (8) et comprenant une couche de support (44),
au moins une section de poche (34) s'étendant dans la direction longitudinale (8) et contiguë à la section de support (36) dans la direction longitudinale (8) et comprenant au moins une poche (22) destinée à recevoir les composants électriques et/ou électroniques (6),
dans lequel ladite au moins une poche (22) comprend une première couche de poche (38) tournée vers le corps et une deuxième couche de poche (40) montrant dans la direction opposée au corps, qui délimitent entre elles un espace de poche (42),
dans lequel ladite au moins une poche (22) présente au moins une ouverture de poche (30) accessible dans la direction transversale (10),
dans lequel le matériau plat textile (2) comprend des fils de chaîne (48) disposés dans la direction longitudinale (8) et des fils de trame (50) disposés dans la direction transversale (10), qui forment la couche de support (44), la première couche de poche (38) et la deuxième couche de poche (40),
dans lequel, dans la direction de l'épaisseur (13) entre la première couche de poche (38) et la deuxième couche de poche (40), au moins un conducteur électrique flexible (46) s'étendant dans la direction longitudinale (8) et disposé au moins par sections de manière flottante est prévu, et dans lequel ledit conducteur électrique (46) disposé de manière flottante présente une distance de flottement (47) d'au moins 5 mm.

2. Matériau plat textile (2) selon la revendication 1, **caractérisé par le fait que** le conducteur électrique (46) disposé de manière flottante présente une distance de flottement (47) d'au moins 10 mm, et/ou de 100 mm tout au plus, en particulier de 50 mm tout au plus, en particulier de 30 mm tout au plus, en particulier de 20 mm tout au plus.

3. Matériau plat textile (2) selon la revendication 1 ou 2, **caractérisé par le fait qu'**une autre section de support (36) est contiguë à la section de poche (34) dans la direction longitudinale (8) en regard de la section de support (36), dans lequel la section de poche (34) est disposée dans la direction longitudinale (8) entre les sections de support (36) et/ou qu'une autre section de poche (34) est contiguë à la section de support (36) dans la direction longitudinale (8) en regard de la section de poche (34), dans lequel la section de support (36) est disposée dans la direction longitudinale (8) entre les sections de poche (34).

4. Matériau plat textile (2) selon l'une quelconque des revendications précédentes, **caractérisé par le fait que** le matériau plat textile (2) est réalisé sans fin dans la direction longitudinale (8), avec une section de support (36) et une section de poche (34) en alternance.

5. Matériau plat textile (2) selon l'une quelconque des revendications précédentes, **caractérisé par le fait que** les fils de chaîne (48) s'étendant dans la direction longitudinale (8) sont réunis à partir de la section de poche (34), dans la transition vers la section de support (36), pour former la couche de support (44).

6. Matériau plat textile (2) selon l'une quelconque des revendications précédentes, **caractérisé par le fait que** ledit au moins un conducteur électrique (46) est disposé sur la couche de support (44), en particulier est entouré en partie par la couche de support (44) ou y est maintenu d'une autre manière.

7. Matériau plat textile (2) selon l'une quelconque des revendications précédentes, **caractérisé par le fait que** plusieurs conducteurs électriques (46) s'étendant pour l'essentiel parallèlement les uns aux autres dans la direction longitudinale (8) sont prévus, dans lequel les conducteurs électriques (46) sont espacés les uns des autres dans la direction transversale (10) de telle manière que, lors d'une utilisation conforme du matériau plat textile (2), il n'y a pas de contact direct entre deux ou plusieurs conducteurs électriques (46).

8. Matériau plat textile (2) selon l'une quelconque des revendications précédentes, **caractérisé par le fait que** ledit au moins un conducteur électrique (46) est isolé au moins par sections dans la section de support (36) et/ou que ledit au moins un conducteur électrique (46) n'est pas isolé ou bien est dénudé au moins par sections dans la section de poche (34) de sorte qu'un contact électrique peut être établi entre ledit au moins un conducteur électrique (46) et le composant électrique et/ou électronique (6).

9. Matériau plat textile (2) selon l'une quelconque des revendications précédentes, **caractérisé par le fait que** ledit au moins un conducteur électrique (46) est séparé dans la section de poche (34) de sorte que ce conducteur électrique (46) présente deux extrémités de conducteur libres (52) en particulier destinées à entrer en contact avec le composant électrique et/ou électronique (6).

10. Matériau plat textile (2) selon la revendication 9, **caractérisé par le fait qu'**un connecteur (54) est prévu sur au moins une extrémité de conducteur libre (52) du conducteur électrique (46) séparé de sorte qu'une connexion mâle-femelle peut être établie avec le composant électrique et/ou électronique (6).

11. Matériau plat textile (2) selon l'une quelconque des revendications précédentes, **caractérisé par le fait que** ladite au moins une poche (22) est fermée en vis-à-vis en réunissant la première couche de poche (38) et la deuxième couche de poche (40) dans la direction transversale (10) de l'ouverture de poche (30).

12. Matériau plat textile (2) selon l'une quelconque des revendications précédentes, **caractérisé par le fait qu'**une zone latérale (32) est contiguë à ladite au moins une poche (22) dans la direction transversale (10) en regard de l'ouverture de poche (30), dans lequel, dans la zone latérale (32), la première couche de poche (38) et la deuxième couche de poche (40) sont reliées l'une à l'autre de manière plane, en particulier dans lequel, dans cette zone latérale (32), les fils de trame (50) s'étendant dans la direction transversale (10) et les fils de chaîne (48) s'étendant dans la direction longitudinale (8) sont tissés entre eux.

13. Matériau plat textile (2) selon l'une quelconque des revendications précédentes, **caractérisé par le fait qu'**un moyen de positionnement (55) destiné à positionner et/ou à arrêter un composant électrique et/ou électronique (6) apte à être inséré dans la poche (22) est prévu sur ou dans la poche (22).

14. Matériau plat textile (2) selon l'une quelconque des revendications précédentes, **caractérisé par le fait que** dans la section de support (36) et/ou dans la section de poche (34) sont prévus des fils de chaîne (48) élastiquement extensibles s'étendant dans la direction longitudinale (8) et/ou des fils de trame (50) élastiquement extensibles s'étendant dans la direction transversale (10).

15. Dispositif de maintien ou de support (60) pour un composant électrique et/ou électronique (6), comprenant:
un matériau plat textile (2) selon une ou plusieurs des revendications précédentes.

16. Dispositif (62) à des fins de mesure et/ou de commande de technique médical destiné à être utilisé sur le corps humain ou animal (4), comprenant un dispositif de maintien ou de support (60) selon la revendication 15 et un composant électrique et/ou électronique (6) maintenu sur celui-ci et contacté électriquement, en particulier un microcontrôleur, une batterie, une batterie rechargeable et/ou un capteur pour détecter des signaux corporels et/ou de mouvement ou un composant pour générer et/ou fournir des signaux électriques au corps (4).
